# EUROPÄISCHE PATENTSCHRIFT

(11) **EP 0 664 704 B1**
(45) Veröffentlichungstag und Bekanntmachung des Hinweises auf die Patenterteilung: **29.04.1998**
(21) Anmeldenummer: 93920662.9
(22) Anmeldetag: 22.09.1993
(51) Int. Cl.: A61K 31/48, A61K 31/00

(54) **VERWENDUNG VON DOPAMINAGONISTEN ZUR AKTIVIERUNG DER GAMMA INTERFERON-PRODUKTION**
USE OF DOPAMINE AGONISTS TO ACTIVATE GAMMA-INTERFERON PRODUCTION
UTILISATION D'AGONISTES DE LA DOPAMINE POUR ACTIVER LA PRODUCTION D'INTERFERON GAMMA

(30) Priorität: 06.10.1992 DE 4234380
(43) Veröffentlichungstag der Anmeldung: 02.08.1995
(73) Patentinhaber: SCHERING AKTIENGESELLSCHAFT, 13353 Berlin (DE)
(72) Erfinder: HOROWSKI, Reinhard, D-13465 Berlin (DE); SUCHY, Irmgard, D-14055 Berlin (DE); PRZUNTEK, Horst, D-44801 Bochum (DE); POEHLAU, Dieter, D-45527 Hattingen (DE); POGGEL, Hans-Arnold, / (DE)
(86) Internationale Anmeldenummer: DE9300911
(87) Internationale Veröffentlichungsnummer: WO9407497

(56) Entgegenhaltungen:
- IMMUNOPHARMACOL. IMMUNOTOXICOL. Bd. 13, Nr. 1-2 , 1991 Seiten 47 - 64 J.L. BUSSIERE ET AL 'Effects of bromocriptine treatment on immune responses and 3-methylcholantrene-induced tumorigenesis in rats.'

## Beschreibung

Die Erfindung betrifft die Verwendung von Dopaminrezeptoragonisten und deren physiologisch verträgliche Salze zur Aktivierung der lokalen Freisetzung von γ-Interferon (IFN-γ) und α-Tumornekrosefaktor (TNF-α).

γ-Interferon wird überwiegend von T-Zellen gebildet, darüber hinaus von "Natural Killer"-Zellen (NK-Zellen) und in selteneren Fällen von Epithelien und Fibroblasten. γ-Interferon hat 3 Hauptwirkungen: antiviral, antiproliferativ und immunmodulierend, wobei die immunmodulierende Wirkung über die Aktivierung der Monozyten/Makrophagen-Aktivität erfolgen kann. Es besteht ein Synergismus mit Interleukin 2 (JL2).

Die Major-Histocompatibilitäts-Komplex-(MHC-)-Antigenexpression der Klasse I und II wird gesteigert, die Zellproliferation wird gehemmt und die Freisetzung anderer Cytokine wird beeinflußt.

Der immunaktivierende, antiproliferative, tumorhemmende Effekt von IFN-γ allein und in Kombination mit anderen Substanzen ist belegt. So konnte die chemisch bedingte Karzinogenese von Mäusen durch die Gabe von IFN-γ verhindert werden (Salerno et al. Nature New Biol. 239, 31-32, 1972). In der Tumorbiologie greift IFN-γ u.a. dadurch ein, daß es die Expression von Protoonkogenen vermindert. Bei Patienten mit Gliomen (wie auch bei Patienten mit anderen Malignomen) ist die IFN-γ Freisetzung nach Stimulation signifikant vermindert. Die verminderte Stimulierbarkeit des IFN-γ ist ein prognostischer Faktor, d.h. Patienten, die vermindert IFN-γ produzieren, haben eine geringere Überlebensdauer. In vitro-Versuche haben eine synergistische oder zumindest additive Wirkung von IFN-γ und herkömmlichen Cytostatika gezeigt. Auch der Anstieg des TNF-α und die Modulation weiterer Cytokine hemmen die Zellproliferation.

Bei einer Reihe von Erkrankungen wurde eine (evtl. primäre) Defizienz von Interferonen gefunden, so beim Down-Syndrom, beim selektiven IgA-Mangel und beim Hyper-IgE-Syndrom. Bei Patienten mit Aids ist die beeinträchtigte IFN-γ-Freisetzung ein prognostisch ungünstiger Faktor, auch bei noch ausreichenden CD4 (Helfer)-Lymphozyten. Auf mitogene Reize sind einige der Patienten in fortgeschrittenen Stadien nicht in der Lage, IFN-γ zu sezernieren. In vitro sind aber die Effektorzellen (z.B. Makrophagen) durch extern zugeführtes IFN-γ stimulierbar und entfalten dann erhöhte antimikrobielle Aktivität.

Der Einsatz von IFN-γ bei nicht malignen Erkrankungen wurde bisher nur im Rahmen kontrollierter Studien wie zum Beispiel bei chronischer Granulomatose durchgeführt. Für verschiedene erregerbedingte Krankheiten wie beispielsweise Leishmaniase und Lepra ist die IFN-γ-Therapie geeignet. Bei der Sklerodermie inhibiert IFN-γ in vitro die Kollagensynthese von Fibroblasten. Bei allergischen Erkrankungen zeigen Studien an Maus-Modellen und in vitro, daß IFN-γ den Effekt von IL-4 auf die IgE Synthese unterdrücken kann. Auch ein Antagonismus zum Il-8 ist beschrieben.

Die systemische Gabe von IFN-γ ist unphysiologisch und wird oft nicht toleriert, denn IFN-γ wirkt physiologischerweise autokrin, parakrin, also direkt vor Ort.

Wenn man bei einem Patienten IFN-γ im Blut (ohne Stimulation) nachweisen kann, dann ist dieser immer schwerkrank. Die systemische, externe Gabe von IFN-γ simuliert eine "endokrine" Bildung, die von der Natur gar nicht vorgesehen ist. Der Körper wird also in hohen Dosen von einer hochwirksamen Substanz überschwemmt. Man wird so trotz hoher systemischer Dosen nur relativ geringe lokale Spiegel erreichen. Außerdem unterliegt die externe Gabe nicht der endogenen Regulation. Deshalb hat die Therapie mit IFN-γ oft gravierende Nebenwirkungen, die einen breiteren Einsatz limitieren: "Grippe-Symptome", Kopf- und Gelenkschmerzen, Schüttelfrost, Fieber, Übelkeit, Erbrechen, Bluthochdruck, Arrhythmien, Tachykardien, Kardiomyopathien, Thrombozytopenien, Leukozytopenien, Leberwertveränderungen.

Es war daher wünschenswert, die endogene Freisetzung von IFN-γ vor Ort durch geeignete und verträgliche Substanzen zu modulieren.

Es wurde nun gefunden, daß Dopamin-Agonisten als Priming Faktor wirken, so daß das Immunsystem bei endogenen oder exogenen Reizen vermehrt IFN-γ und andere Mediatoren zur Verfügung stellt.

Die vorliegende Erfindung umfaßt daher die Verwendung von Dopaminagonisten zur Aktivierung der lokalen Freisetzung von γ-Interferon und α-Tumornekrosefaktor in Lebewesen dadurch, daß man Dopaminagonisten in verschiedener Weise systemisch oder lokal appliziert.

Als Dopaminagonisten sind Ergot-Derivate und als deren Teilstrukturen Chinolinderivate und deren physiologisch verträgliche Salze geeignet. Als Chinolinderivate seien beispielsweise die in EP-A-77754 beschriebenen Verbindungen genannt wie Quinagolid (N,N-Diethyl-N'-[(3R',4aR',10aS')-1,2,3,4,4a,5,10,10a-octahydro-6-hydroxy-1 propyl-3-benzo[g]quinolinyl]-sulfamid).

Als Ergot-Derivate seien Ergolinderivate genannt, die in 8-Stellung α- oder β-substituiert sind, in 8,9- oder 9,10-Stellung eine Einfach- oder Doppelbindung haben, in 6-Stellung mit C₁₋₆-Alkyl substituiert sind und in 2-Stellung mit Halogen, Thiomethyl oder Methyl substituiert sein können. Als Substituent in 8-Stellung ist die Aminogruppe CH₃, CH₂CN, CH₂OH, CH₂-O-C₁₋₆-Alkanoyl und CO-NH-Phenyl geeignet, wobei die Aminogruppe als Harnstoff-, Acylamino- oder Sulfamoylaminoderivat vorliegen kann. Beispielsweise seien genannt Lisurid, Tergurid, Pergolid, Protergerid, Cabergolin und die in EP-A-429153, WO 90/12796 und WO 90/13550 beschriebenen Verbindungen.

Als besonders bevorzugt sind die Verbindungen der Formel I und deren Säureadditionssalze zu betrachten 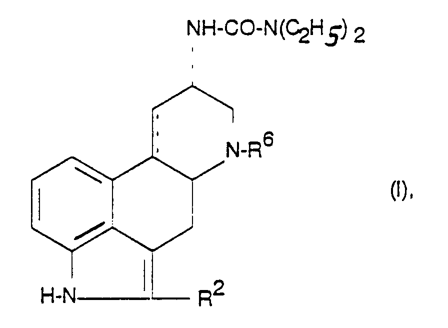 worin
- R⁶: C₁₋₆-Alkyl,
- R²: Wasserstoff, Halogen, SCH₃ oder Methyl und
- C.....C: eine Einfach- oder Doppelbindung ist.

Lisurid und Tergurid haben sich als besonders gut geeignet und verträglich für die erfindungsgemäße Verwendung erwiesen.

Von den genannten Verbindungen ist bekannt, daß sie als potente Dopaminagonisten die Prolactin-Sekretion hemmen und als Antiparkinsonmittel verwendet werden können. Bei Patienten mit Morbus Parkinson finden sich weniger bösartige Tumore als in der Normalbevölkerung (Janson et al., Ann. Neur. 17/5: 505 (1985)). Dies kann möglicherweise zum Teil auf die dopaminerge Behandlung zurückgeführt werden. So ist die Mortalität von Patienten mit Morbus Parkinson unter einer Kombinationstherapie von L-Dopa und Bromocriptin oder Lisurid geringer als unter L-Dopa-Monotherapie. Dem entspricht die Beobachtung, daß die chronische Gabe von z.B. Lisurid, aber auch Tergurid, am Tier geeignet war, das Auftreten von Tumoren nicht nur endokriner Organe (Hypophyse, Mamma, Ovar), sondern auch von anderen Systemen (z.B. Leukämien und Lymphome) in ihrer Häufigkeit und Schwere zu mindern. Auch immunologische Auffälligkeiten werden bei dopaminerg behandelten Patienten in Morbus Parkinson beschrieben. Beispielsweise ist die IgG-Produktion nach mitogener Stimulation bei Parkinson-Patienten vermindert (Martilla et al., Neurol. Sci. 69, 131 (1985)).

Daß bei der Behandlung mit einem Dopaminergikum ein "Priming Effekt" erzeugt wird, der dann bei mitogener Stimulation beipsielsweise mit Phythämagglutimin (PHA) eine vermehrte Freisetzung von IFN-γ und eine Beeinflussung der Interleukine bewirkt, wird am Beispiel von Lisurid gezeigt.

Vergleich einer Lisurid-behandelten Gruppe von Patienten mit M. Parkinson bzw. mit Dystonie mit einer altersgleichen Gruppe Gesunder:

### Material und Methode:

Wir untersuchten die mitogen-induzierte Zytokinfreisetzung von 18 Patienten, die wegen M. Parkinson (n=13) oder Dystonie (n=5) mit subkutanem Lisurid behandelt wurden.
18 Patienten wurden mit 11 gesunden altersgleichen Kontrollen verglichen. Nach mitogener Stimulation mit 50 µl Phythämagglutinin (PHA) (5 µg/ml) über 24 h (TNF-α) bzw. 72h (IFN-γ) wurden TNF-α und IFN-γ im Überstand mit einem Elisa Assay (Gallati 1987) bestimmt:
Da die Daten nicht normal verteilt sind, wurde der Mann-Whitney-Test zur Analyse der Gruppenmittelwerte gerechnet. Es fanden sich folgende Ergebnisse:

Die IFN-γ Freisetzung nach PHA-Stimulation ist bei der Lisurid behandelten Gruppe signifikant erhöht (p=0.039).

Die TNF-α Freisetzung ist deutlich erhöht, (p=0.055).

### Vergleich der Lisurid-behandelten Gruppe mit einer Gruppe junger Gesunder und mit einer Gruppe älterer Gesunder

### Material und Methode:

Es wurde dieselbe Lisurid-Gruppe (n=18) wie o.a. mit zwei Gruppen gesunder Freiwilliger verglichen:
Einer Gruppe junger (Alter unter 50 Jahre) und mit einer Gruppe älterer Gesunder (Alter über 65 Jahre).
Die Zytokinmessung und statistische Auswertung erfolgte wie oben erwähnt. Ergebnisse:
Die IFN-γ Produktion und die α-TNF Produktion nach mitogener Stimulation war bei der Lisurid-Gruppe (Altersmedian 61 J) gegenüber der Gruppe junger und der Gruppe älterer Gesunder signifikant erhöht. Dies legt den Schluß nahe, daß die alterskorreliert abnehmende IFN-γ und TNF-α Produktion durch dopaminerge Therapie antagonisiert werden kann.

Die Aktivierung der Freisetzung von IFN-γ bei dopaminerger Therapie wird auch durch die Beobachtung gestützt, daß bei Überdosierungen an Probanden grippeähnliche Symptome und Myalgien auftreten können.

### In vitro Vergleich der Zytokin-Freisetzung bei Koinkubation mit Lisurid

### Material und Methode:

Von 10 gesunden jungen Freiwilligen wurden Vollblut-Assays (s.o.) mit 4 verschiedenen Konzentrationen von Lisurid zusammen mit dem Stimulans (PHA) inkubiert. Es fanden sich keine Unterschiede zwischen den Gruppen ohne Lisurid, mit geringer, therapeutischer und (beim Menschen) toxischer Lisuridkonzentration. Dies läßt vermuten, daß es sich bei den Patientendaten nicht um kurzzeitige Effekte der Substanz auf die immunkompetenten Zellen (vor allem T-Lymphozyten und NK-Zellen) handelt. Vielmehr werden über Rezeptor/postrezeptorische Mechanismen Effekte auf die Freisetzung und/oder Synthese von IFN-γ und anderen Faktoren vermittelt. Auch ein direkter oder indirekter Effekt auf das Genom ist möglich. Für die therapeutische Anwendung erwünscht ist die beobachtete Anreicherung von Dopaminagonisten in den Membranen von weißen Blutzellen, wie sie im Falle von Lisurid durch die UV-Fluoreszenz dieses Moleküls nachweisbar ist.

In vivo-Untersuchungen an Nager-Gruppen von männlichen und weiblichen Mäusen und Ratten wurden mit verschiedenen Dosen von Lisurid und Tergurid über 2 Jahre behandelt. Hierbei fand sich eine zum Teil dosis-abhängige, signifikante Reduktion der Häufigkeit und Schwere von verschiedenen Tumoren wie z.B. Leukämien und Lymphome sowie von Tumoren der Mammae, der Hypophyse und der Ovarien und hiermit verbunden eine signifikante Erhöhung des Anteils überlebender Tiere durch diese dopaminerge Behandlung. Untersuchungen am Affen mit extrem hoher parenteraler Applikation von Lisurid (1 mg/kg/Tag s.c. über 6 Monate kontinuierlich infundiert, d.h. die 10-fache maximale Humandosis) haben weiterhin Befunde ergeben, wie sie für eine Überproduktion oder Freisetzung von IFN-γ und vergleichbarer Substanzen typisch sind. Dies läßt eine therapeutisch erwünschte lokale Freisetzung dieser Faktoren bei niederer Dosierung von Dopaminagonisten erwarten, die erst bei excessiver Dosierung zur Ausschwemmung ins Blut gelangt und damit die Nebenwirkung einer direkten IFN-γ-Anwendung bewirkt.

Den Daten ist zu entnehmen, daß durch die Behandlung mit Lisurid nach Stimulation eine vermehrte Freisetzung von IFN-γ und TNF-α erfolgt. Die Freisetzung der Cytokine ist erst in vitro in einer Kurzzeit-Kultur des Vollblutes entstanden. In der Kontrollgruppe ohne PHA Zugabe ließ sich kein IFN-γ nachweisen. In vitro werden dabei die immunkompetenten Zellen durch Mitogene stimuliert und setzen dabei die Cytokine frei. In vivo findet diese Stimulation durch das spezifische Antigen statt. Die in vitro erhöhte Cyctokinfreisetzung in immunkompetenten Zellen von Patienten, die mit Lisurid behandelt wurden, zeigt, daß über einen Priming Effekt die Potenz der Zellen zur Cytokinfreisetzung "auf Anforderung" erhöht wird. Hieraus läßt sich schließen, daß der erwünschte Effekt der IFN-γ-Freisetzung lokalisiert und reguliert erfolgt.

Auf Grund ihrer Aktivität die IFN-γ Produktion zu steigern, können Dopaminagonisten verwendet werden zur Behandlung und Prophylaxe von Krankheiten, die eine regulierte Steigerung der IFN-γ- und der TNF-α-Produktion und der Modifikation der Interleukine erfordern und direkt oder indirekt über diese Mediatoren zu beeinflussen sind.

Dopaminagonisten eignen sich daher zur Behandlung von Krankheiten, bei welchen eine Freisetzung und Aktivierung von IFN-γ ,TNF-α und ähnlichen Mediatoren erwünscht und zweckmäßig ist, bevorzugt, wenn eine systemische Applikation dieser Faktoren vor Ort nicht wirksam wird oder zu viele Nebenwirkungen auslöst. So ist bereits bekannt, daß IFN-γ bei chronisch entzündlichen Erkrankungen wie Polyarthritis und chronischer Granulomatose wirksam ist, daß aber die systemische Verabreichung eine Vielzahl teilweise gravierender lokaler und generalisierter Nebenerscheinungen auslöst; hierzu gehören z.B. Dermatitis, Haarausfall, Neuralgien, Durchblutungsstörungen, Funktionsstörungen der Leber, lebensbedrohliche Störungen des Blutbildes sowie lokale Unverträglichkeiten an der Injektionsstelle. Die indirekte Aktivierung von IFN-γ und anderen Faktoren durch Dopaminagonisten ist dazu geeignet, diese systemischen und lokalen Intoleranzerscheinungen zu vermeiden oder in ihrer Häufigkeit und Schwere deutlich zu verringern.

Die Aktivierung des Immunsystems und besonders der Effekt einer lokalen Freisetzung von IFN-γ ist weiterhin geeignet die zelluläre Abwehr gegenüber unterschiedlichen Infektionen zu verstärken wie z.B. bei Tuberkulose, Leishmaniose, Toxoplasmose, Rickettsiosen und andere Erkrankungen und Infektionen, bei welchen eine Aktivierung der Makrophagenaktivität erwünscht ist.

Eine solche über IFN-γ und vergleichbare Faktoren vermittelte Aktivierung zellulärer Immunität betrifft auch das monocyten-makrophagocytäre System sowie besonders die Natural-Killer-Zellen, deren Aktivierung insbesondere bei Tumorerkrankungen erwünscht ist und zwar sowohl bei Tumoren solider Organe als auch bei Leukämien und vergleichbaren Erkrankungen. Eine tumorhemmende Wirkung durch IFN-γ als Folge vermehrter Freisetzung durch dopaminerge Therapien wird in vielen Fällen noch gefördert und verstärkt, da die hier beschriebenen Substanzen auch die Freisetzung von TNF-α fördern. Auch hier lassen sich die systemischen Begleiterscheinungen dieser Therapie durch die indirekte, auf die lokale Freisetzung der Mediatoren zielende Anwendung von Dopaminergika vermeiden oder vermindern.

IFN-γ wirkt antagonistisch zu Interleukin-4. Dieses Cytokin spielt eine zentrale Rolle bei atopischen, allergischen Erkrankungen. Eine Erhöhung der IFN-γ Freisetzung führt zu einer Herabregulation der Il-4 Synthese. Daher können die hier beschriebenen Substanzen auch zur Behandlung atopischer, allergischer Erfkrankung sowie als Adjuvans zu bekannten Therapieprinzipien eingesetzt werden.

Aufgrund der erfindungsgemäßen Wirkung eignen sich Dopaminagonisten zur Behandlung der oben genannten Krankheiten wie zur Behandlung von Infekten wie beispielsweise bei Schwerbrandverletzten, zur Behandlung chronisch entzündlicher Erkrankungen wie Arthritis und andere rheumatische Erkrankungen, sowie andere Erkrankungen mit Granulomen, Psoriasis, Lupus Erythematodes, Sklerodermie, Störungen der Immun- und Abwehrfunktion beim Down-Syndrom oder bei Aids, Störungen der Immun- und Abwehrfunktion unter Chemotherapie oder sonstiger immun-suppressiver Medikation sowie zur Behandlung von Tuberkulose und anderen bakteriellen, viralen und parasitäten Erkrankungen wie Leishmaniose, Lepra sowie zur Behandlung neoplastischer und atopischer Erkrankungen.

Die Arzneimittel werden nach an sich bekannten Verfahren hergestellt, indem man den Wirkstoff mit geeigneten Träger-, Hilfs- und/oder Zusatzstoffen in die Form eines pharmazeutischen Präparates bringt, das für die enterale oder parenterale Applikation geeignet ist. Die Applikation kann oral, transdermal oder sublingual, als Feststoff in Form von Kapseln oder Tabletten oder als Flüssigkeit in Form von Lösungen, Suspensionen, Elixieren oder Emulsionen oder rektal in Form von Suppositorien oder in Form von gegebenenfalls auch subcutan anwendbaren Injektionslösungen erfolgen. Auch eine lokale Applikation in Form von Pellets ist möglich. Als Hilfsstoffe für die gewünschte Arzneimittelformulierung sind die dem Fachmann bekannten inerten organischen und anorganischen Trägermaterialien geeignet wie zum Beispiel Wasser, Gelantine, Gummi arabicum, Milchzucker, Stärke, Magnesiumstearat, Talk, pflanzliche Öle, Polyalkylenglykole usw.. Gegebenenfalls können darüberhinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form zum Beispiel als Tabletten, Dragees, Suppositoren, Kapseln, Salben oder in flüssiger Form zum Beispiel als Lösungen, Suspensionen oder Emulsionen vorliegen oder als Depotzubereitung formuliert sein.

Als Trägersysteme können auch grenzflächennahe Hilfsstoffe wie Salze der Gallensäure oder tierische oder pflanzliche Phospholide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie z.B. Lactose, Mais- oder Kartoffelstärke, geeignet. Die Anwendung kann auch in flüssiger Form erfolgen, wie z.B. als Saft, dem gegebenenfalls ein Süßstoff beigefügt wird.

Die Dosierung der Wirkstoffe kann je nach Verabfolgungsweg, Alter und Gewicht des Patienten, Art und Schwere der zu behandelnden Erkrankung und ähnlichen Faktoren variieren. Im allgemeinen werden gute Ergebnisse erzielt, wenn man bei chronischer Anwendung eine Dosis, die zu einer deutlichen und zuverlässigen Senkung des peripheren Prolactinspiegels führt oder ein Vielfaches (bis maximal 10-faches) davon appliziert. Die tägliche Dosis beträgt 0,001 - 10 mg, wobei die Dosis als einmal zu verabreichende Einzeldosis oder unterteilt in 2 oder mehrere Tagesdosen gegeben werden kann, entsprechend den wirkäquivalenten Dosen der Substanzen. Die Wirkstoffe können auch mit anderen Therapie-Maßnahmen (zum Beispiel Antibiotika, Chemotherapie, chirurgische Maßnahmen) kombiniert werden.

## Patentansprüche

1. Verwendung von Dopaminagonisten zur Herstellung eines Arzneimittels zur lokalisierten und regulierten Freisetzung von IFN-γ und TNF-α zur Behandlung von Krankheiten, die über IFN-γ und TNF-α beeinflußt werden.

2. Verwendung von Dopaminagonisten zur Herstellung eines Arzneimittels zur lokalisierten und regulierten Freisetzung von IFN-γ zur Behandlung von Krankheiten, die durch Störungen der Freisetzung von IFN-γ ausgelöst werden.

3. Verwendung nach Anspruch 1 und 2, dadurch gekennzeichnet, daß eine normale oder verminderte Immun- und Abwehrfunktion verstärkt wird.

4. Verwendung nach Anspruch 1 bis 3, dadurch gekennzeichnet, daß die Makrophagenaktivität erhöht wird.

5. Verwendung nach Anspruch 1 bis 3, dadurch gekennzeichnet, daß die Il-4 Synthese vermindert wird.

6. Verwendung nach Anspruch 1 bis 5, dadurch gekennzeichnet, daß parasitäre, bakterielle oder virale Infektionen oder neoplastische Erkrankungen behandelt werden.

7. Verwendung nach Anspruch 1 bis 5 dadurch gekennzeichnet, daß chronisch entzündliche Erkrankungen, atopische Erkrankungen, Erkrankungen mit Granulomen, Psoriasis, Lupus Erythematodes, Sklerodermie, Tuberkulose, oder Störungen der Immun- und Abwehrfunktion beim Down-Syndrom oder bei Aids behandelt werden.

8. Verwendung von Dopaminagonisten nach Anspruch 1 bis 7, wobei der Dopaminagonist eine Verbindung der Formel oder deren Säureadditionssalze 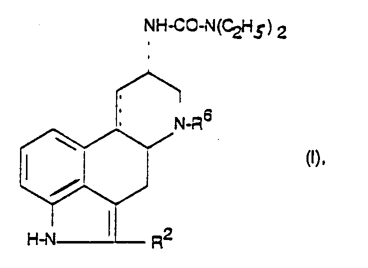 worin
R⁶ C₁₋₆-Alkyl,
R² Wasserstoff, Halogen, SCH₃ oder Methyl und
C.....C eine Einfach- oder Doppelbindung ist, bedeutet.

9. Verwendung von Dopaminagonisten nach Anspruch 1 bis 7, wobei der Dopaminagonist Lisurid oder Tergurid oder deren physiologisch verträgliches Salz ist.

## Claims

1. Use of dopamine agonists for the preparation of a medicament for the localised and regulated release of IFN-γ and TNF-α for the treatment of diseases that are influenced *via* IFN-γ and TNF-α.

2. Use of dopamine agonists for the preparation of a medicament for the localised and regulated release of IFN-γ for the treatment of diseases that are caused by disturbances in the release of IFN-γ.

3. Use according to claim 1 and 2, characterised in that a normal or reduced immune and defence function is strengthened.

4. Use according to claim 1 to 3, characterised in that macrophage activity is increased.

5. Use according to claim 1 to 3, characterised in that IL-4 synthesis is reduced.

6. Use according to claim 1 to 5, characterised in that parasitic, bacterial or viral infections or neoplastic diseases are treated.

7. Use according to claim 1 to 5, characterised in that chronically inflammatory diseases, atopic diseases, diseases with granulomas, psoriasis, lupus erythematosus, sclerodermia, tuberculosis, or disorders of the immune and defence function in the case of Down's syndrome or in the case of AIDS, are treated.

8. Use of dopamine agonists according to claim 1 to 7, wherein the dopamine agonist is a compound of the formula 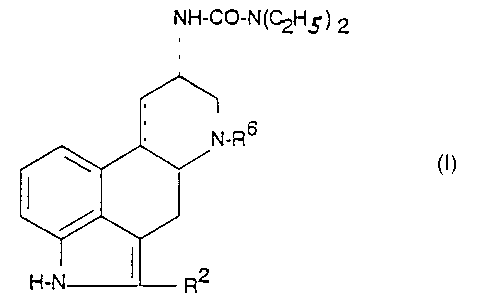 or an acid addition salt thereof,
in which
R⁶ is C₁₋₆alkyl,
R² is hydrogen, halogen, SCH₃ or methyl, and
C.....C is a single bond or a double bond.

9. Use of dopamine agonists according to claim 1 to 7, wherein the dopamine agonist is lysuride or terguride or a physiologically tolerable salt thereof.

## Revendications

1. Utilisation d'agonistes de la dopamine pour préparer un médicament destiné à la libération localisée et régulée de l'IFN-γ et du TNF-α, pour le traitement de maladies influencées par l'IFN-γ et le TNF-α.

2. Utilisation d'agonistes de la dopamine pour préparer un médicament destiné à la libération localisée et régulée de l'IFN-γ, pour traiter des maladies qui sont déclenchées par des troubles de la libération de l'IFN-γ.

3. Utilisation selon les revendications 1 et 2, caractérisée en ce que les fonctions immunitaires et défensives, normales ou réduites, sont intensifiées.

4. Utilisation selon les revendications 1 à 3, caractérisée par une augmentation de l'activité des macrophages.

5. Utilisation selon les revendications 1 à 3, caractérisée par une diminution de la synthèse de l'IL-4.

6. Utilisation selon les revendications 1 à 5, caractérisée par le traitement d'infections parasitaires, bactériennes ou virales, ou de maladies néoplasiques.

7. Utilisation selon les revendications 1 à 5, caractérisée par le traitement de maladies inflammatoires chroniques, de maladies atopiques, de maladies avec des granulomes, du psoriasis, du lupus érythémateux, de la sclérodermie, de la tuberculose ou des troubles de la fonction immune et de défense dans le syndrome de Down ou dans le sida.

8. Utilisation d'agonistes de la dopamine selon les revendications 1 à 7, dans laquelle l'agoniste de la dopamine est un composé ayant la formule suivante, ou l'un de ses sels d'addition avec un acide, 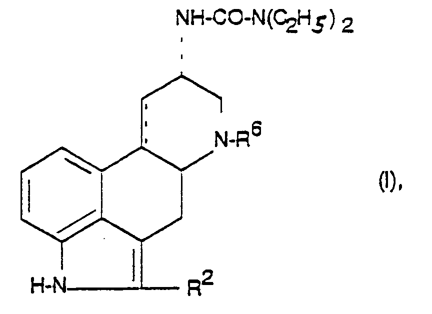 où :
R⁶ est un groupe alkyle en C₁₋₆,
R² est un atome d'hydrogène ou d'halogène ou le groupe SCH₃ ou méthyle,
et
C.....C est une liaison simple ou double.

9. Utilisation agonistes de la dopamine selon les revendications 1 à 7, dans laquelle l'agoniste de la dopamine est le lisuride ou le terguride, ou l'un de ses sels acceptables d'un point de vue physiologique.
